# EUROPEAN PATENT APPLICATION

(11) **EP 3 716 284 A1**
(43) Date of publication of application: **30.09.2020**
(21) Application number: 19166218.8
(22) Date of filing: 29.03.2019
(51) Int. Cl.: G16H 50/30, A61B 5/00, G06N 3/00

(54) **SYSTEM FOR ASCERTAINING A VELOCITY WITHIN TISSUE AND USE THEREOF IN TISSUE TREATMENT**

(71) Applicant: Koninklijke Philips N.V., 5656 AG Eindhoven (NL)
(72) Inventor: VAN SLOUN, Ruud Johannes Gerardus, 5656 AE Eindhoven (NL); BELT, Harm Jan Willem, 5656 AE Eindhoven (NL)
(74) Representative: de Haan, Poul Erik

(57) **Abstract**

This invention relates to an apparatus and a system comprising the apparatus for ascertaining velocities or strain rate values for various depths within a tissue by a neural network based on a plurality of received radiofrequency or in-phase and quadrature (IQ) demodulated signals, indicative of reflected echoes from various depths of the tissue over time. The invention further relates to use of the apparatus and system for tissue treatment.

## Description

### FIELD OF THE INVENTION

This invention relates to an apparatus and a system for ascertaining a velocity within tissue. The invention further relates to use of the apparatus and system for tissue treatment.

### BACKGROUND OF THE INVENTION

US 2017/0311922 A1 discloses a visualization apparatus comprising a signal processor for processing measurement signals from an ultrasound measurement and a rendering device coupled to a processor for rendering a representation for discerning a region of tissue with changed property upon energy application to the tissue from a region with unchanged property within two extremities of the representation indicative of two boundaries defining the tissue thickness, wherein the property of the tissue is a physical quantity selected from a group consisting of velocity, velocity gradient and strain rate.

### SUMMARY OF THE INVENTION

It is an object of the present invention to provide a system with improved performance for ascertaining velocities at various depths of the tissue and/or facilitating detection of the boundary between treated and non-treated portions of tissue based on changes of velocities within tissue.

In a first aspect of the invention, a system for ascertaining a property within a tissue is provided, wherein the system comprises a processor configured to:
receive a plurality of radiofrequency (RF) or in-phase and quadrature (IQ) demodulated signals, wherein said plurality of signals are indicative of reflected echoes from various depths of the tissue over time;
ascertain velocities or strain rate values for various depths of the tissue from said plurality of signals by using a neural network.

The displacement and velocity of a medium can be ascertained from ultrasound radiofrequency or IQ demodulated data. Typically, such processing comprises calculation of autocorrelation functions across an ensemble of consecutive RF or IQ signals. Processors applying 'Kasai' and 'Loupass' autocorrelators for Doppler technique based blood flow calculation are disclosed in C. Kasai, et.al. "Real-Time Two-Dimensional Blood Flow Imaging Using an Autocorrelation Technique" 1985, and in T. Loupas, et.al. "An axial velocity estimator for ultrasound blood flow imaging, based on a full evaluation of the Doppler equation by means of a two-dimensional autocorrelation approach," IEEE Trans. Ultrason. Ferroelectr. Freq. Control, vol. 42, no. 4, pp. 672-688, Jul. 1995.

The velocities ascertained by using a neural network can be rendered on a display of the system. As it will be described in the detailed description section, the system wherein the velocities for various depths of the tissue are ascertained by using a neural network, presents significant improvement with respect to lower noise levels and reduced artifacts (ring-down of the transducer signal, clear demarcation of the back-wall of the tissue) with respect to Kasai's method.

The neural network can be used to perform Doppler imaging from compressed acquisitions, by efficiently encoding relevant information into a latent feature space and decoding this as Doppler estimates. By tweaking the convolution strides, down-sampling factors, and (feature) channels in the encoder network, data compression rates between 32:1 - 128:1 can easily be reached; wherein higher rates lead to an increased degree of spatial smoothness and, interestingly, significant noise suppression. The latter is a direct consequence of the inability of expressing signals with such high entropy in the compact basis of the encoder. The achievable compression rates for 2D and 3D ultrasound data is even higher than for motion-mode ultrasound data, as there is more spatiotemporal redundancy to exploit.

The ability to perform Doppler using a neural network opens up new possibilities for learning dedicated data compression schemes to reduce the bandwidth for probe-scanner communication.

The system may further comprise an energy source connected to an energy application device for applying energy to the tissue so as to change the property of the tissue, and an ultrasound measurement arrangement, wherein the system is arranged to discontinue transmission of energy from the energy source to the energy application device in response to a detected progression of change of velocities and/or strain rate values in the depth of the tissue, derived by the processor based on the ascertained velocities for the various depths of the tissue.

Due to the lower noise levels in the ascertained velocities at various depths of the tissue, the detection of the progression of change of velocities within the tissue (or strain rate values within the tissue derived from velocities) upon application of energy to the tissue for treatment purposes is significantly improved. Another significant improvement is attributed to the reduced level of artifacts, especially for the improved demarcation of the back-wall of a tissue, based on computations using the neural network, as for various applications of tissue treatment by energy (e.g. ablation energy) it is essential to determine the endpoint of the treatment and when energy application to the tissue should be ceased. For treatment of cardiac tissue it is important to apply ablation energy until the property of the tissue upon ablation is changed up to the back-wall of the tissue, and to discontinue immediately the energy application in order to avoid damaging potential adjacent anatomical structures, such as lung tissue or esophagus. Similarly, for treatment of tumors in other organs or ablation of anatomical structures such as vessels, a clear delimitation of boundaries between various structures of anatomies is of paramount importance.

In an aspect of the invention a method of ascertaining a property within a tissue is provided, the method comprising:
receiving a plurality of radiofrequency or in-phase and quadrature (IQ) demodulated signals, wherein said plurality of signals are indicative of reflected echoes from various depths of the tissue over time;
ascertaining velocities or strain rate values at various depths of the tissue from said signals by using a neural network.

The velocities and/or strain rate within the tissue is directly linked to property of the tissue, e.g. homogeneity or inhomogeneity of tissue, behavior upon internal or external stimulus such as heart beat, breathing or palpation, structural change of the tissue due to treatment by chemical compound or heat application, structural change due to functional anomalies of the tissue such as partial infarction of heart tissue or tumor detection in tissue of various organs.

Likewise, the system and the method is beneficial for detecting delimitations of various types of tissues, such as cardiac tissue from either of pericardial sac, lung tissue, esophagus, since heat treatment of cardiac tissue for various clinical conditions (e.g. atrial fibrillation, atrial flutter, segmentation of myocardial infarction portions of tissues) should be limited to the treatment of cardiac tissue, without affecting tissue adjacent to the cardiac tissue. An example of such system and method is cardiac ablation monitoring with a catheter comprising radiofrequency ablation electrode and integrated ultrasound transducers in the distal tip of the catheter, with which simultaneous radiofrequency ablation and lesion monitoring can be performed, however, when the catheter is pushed in contact with the endocardial tissue, in the rather dynamic environment due to heart beating and breathing, the cardiac tissue is pushed in contact with the pericardial sac, and adjacent tissue, which is either lung tissue or esophagus. The different types of tissues as subsequent tissue layers present different properties, which can be emphasized by calculation and presentation of velocities or strain rate values at various depths of the tissue.

The method or other preferred embodiments of the method listed in the claims may have similar advantages as those described above for the embodiments of the systems.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of the invention.
Fig. 2A shows tissue velocity estimates in the radial direction across space at a specific time instance, obtained with the exemplary embodiment according to the invention.
Fig. 2B shows tissue velocity estimates in the radial direction along a specific angular line (indicated by dashed line in Fig. 2A) over time.
Fig. 3A shows motion-mode ultrasound image of left-atrial cardiac tissue.
Fig. 3B shows estimation of velocities for various depths of the tissue by using the Kasai autocorrelator, corresponding to Fig. 3A.
Fig. 3C shows ascertained velocities for various depths of the tissue by using neural network, corresponding to Fig. 3A.
Fig. 4 shows schematically and exemplarily an embodiment of an apparatus according to the invention.
Fig. 5 is a schematic and exemplarily embodiment of a system according to the invention.
Fig. 6 shows schematically and exemplarily a diagram of the functional use of the system.
Fig. 7 show exemplarily the change of velocities within tissue upon tissue treatment by ablation.

### DETAILED DESCRIPTION OF EMBODIMENTS

### I. General considerations

Catheter ablation is an effective technique for the treatment of a wide variety of arrhythmias, and relies on the formation of transmural lesions in the myocardium. Achieving transmurality in a safe manner is challenging for the operator, in particular due to lack of adequate feedback on lesion progression and wall thickness during the procedure. Recently, near-field motion-mode ultrasound imaging at the catheter tip was proposed as a solution to enable real-time visualization of lesion formation and determination of transmurality, D.E. Haines, et.al. "Near-field ultrasound imaging during radiofrequency catheter ablation: tissue thickness and epicardial wall visualization and assessment of radiofrequency ablation lesion formation and depth", Circulation: Arrhythmia and Electrophysiology, 10(12):e005295, 2017.

Axial tissue Doppler processing was exploited to measure myocardial strain rate and tissue velocities, which turned out particularly useful for detecting the border between the epicardium and parietal pericardium, as well as for monitoring lesion development through assessment of tissue stiffening.

The design of Doppler estimators for such an application involves careful optimization of the slow- and fast-time range gates, amounting to a trade-off between the signal quality and spatiotemporal resolution. Achieving the desired spatiotemporal resolution to capture the relevant myocardial dynamics as well as reaching a high estimation accuracy and precision therefore leads to optimal settings that can vary across measurements and desired clinical objectives.

From a hardware perspective, a significant challenge for the design of catheter transducers is coping with the limited cable bandwidth and related connectivity constraints. While this is less of a concern for transducers with only few elements, the number of transducer elements have expanded greatly in recent intra cardiac echography devices to facilitate 2D or even 3D imaging. Connectivity and bandwidth challenges are addressed through application-specific integrated circuits (ASICs) that perform microbeamforming to compress the full channel data into a manageable quantity. A natural question that arises is how, and by how much, can these signals be compressed more effectively for retaining sufficient information to perform e.g. reliable Doppler reconstruction? In a preferred embodiment, this invention is an end-to-end learning approach for ascertaining velocity estimates from motion-mode ultrasound channel data, by learning a set of nonlinear operations that can approximate the behavior of the Kasai auto-correlator. In another embodiment, said end-to-end learning approach is used for ascertaining velocity estimates from any ensemble of slow-time ultrasound channel signals. Convolutional networks are used based on an encoder-decoder architecture to learn Doppler features at a hierarchy of scales and abstractions, while the use of small latent bottleneck space inherently de-noises the estimates and compactly compresses the relevant information. The data acquisition protocol is given in Section II-A, after which the adopted network architecture and training strategy are presented in Sections II-B and II-C, respectively. The results are described in Section III.

### II A. Data acquisition

In-vivo open-chest intra-cardiac echography in the right atrium (anterior) of a porcine model has been performed, under a protocol approved by the Internal Committee Biomedical Experiments (ICBE), and the institutional animal experiment committee (DEC) Utrecht, using a linear array miniTEE s7-3t transducer in combination with a Verasonics Vantage system (Kirkland, WA). The transducer had 48 elements and a pitch of 0.151 mm, transmitting and receiving at a center frequency of 4.8 MHz. A 13-angle diverging wave scheme was used. The received radio frequency (RF) data was sampled at a rate of 19.2 MHz, and coherently compounded frames were acquired at a frame rate of 474 kHz. The RF data was subsequently demodulated into its in-phase and quadrature (IQ) components, from which the reference Doppler shifts were obtained through the use of the Kasai auto-correlation technique.

In a different setting, we performed near-field motion-mode intra-cardiac echography of the left-ventricular apex of several canines, under a protocol approved by the Institutional Animal Care and Use Committee of Surpass-Silicon Valley, LLC (Mountain View, CA), using an 8F catheter with 4 ultrasound transducers at the tip (1 axial and 3 radial). These single element transducers had a center frequency of 30 MHz, and the received radio frequency (RF) data was sampled at a rate of 100 MHz. RF lines were acquired at a line rate of 1 kHz. The RF data was subsequently demodulated into its in-phase and quadrature (IQ) components, from which the reference Doppler shifts were obtained through the use of the Kasai auto-correlation technique.

### II B. Deep neural network architecture

This section explains the network architecture that was used to mimic the Kasai Doppler functionality. A fully convolutional neural network was adopted, consisting of an encoder and a decoder. The encoder aims at efficiently mapping a set of 256 sequential input IQ lines into a compact feature representation. To this end, it comprises a series of three identical blocks, each composed of two subsequent two-dimensional convolutional layers that extract features across fast- and slow-time, followed by a spatiotemporal down-sampling operation (max pool) along both dimensions to compress this feature representation. The encoded IQ data was then decoded into Doppler data through a similar set of nonlinear operations, mirroring the structure of the encoder and replacing the down-sampling operations by nearest neighbor up-sampling. All convolutions were followed by leaky rectified linear activation units (leaky ReLUs), except for the final layer, which had no activation function. A batch normalization step has been added after each activation and incorporated dropout to improve training convergence.

### II C. Training strategy

High network generalization and robustness was attained by incorporating 50% dropout at the latent feature layer during training, effectively forcing the network to act as an ensemble of multiple models by randomly disabling nodes with a 50% probability in each iteration. Assuming normally-distributed prediction errors, the network was trained by minimizing the mean squared error between the target Kasai Doppler shifts and the network predictions. To this end, an Adam optimizer (D.P. Kingma, J.L. Ba, "ADAM: A method for stochastic optimization, ICLR 2015, p. 1-15, https://arxiv.org/pdf/1412.6980") was employed with a learning rate of 0.001, stochastically optimizing on mini-batches consisting of 16 sets of 256 sequential IQ lines.

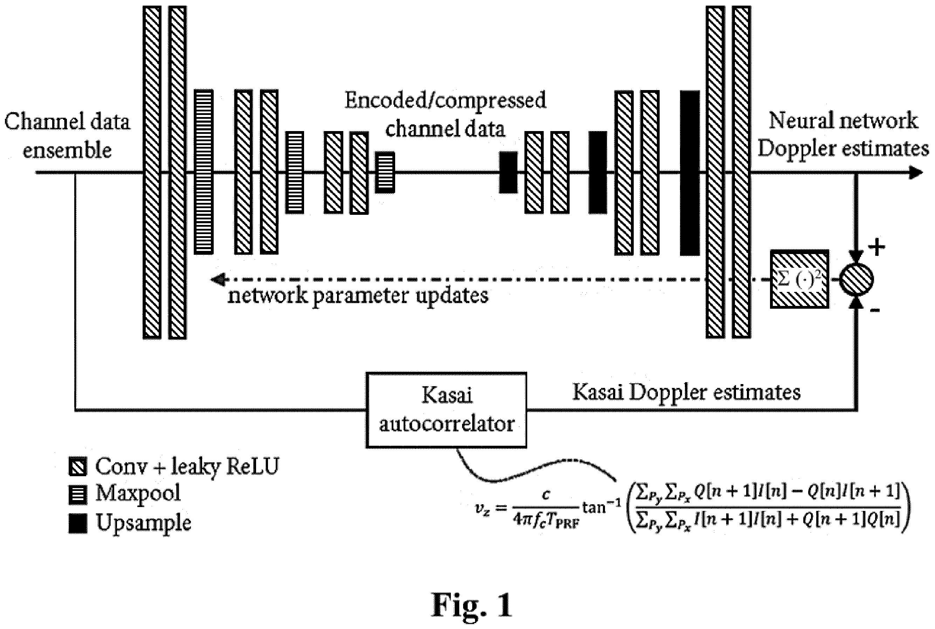

An overview of the neural network architecture and training strategy is given in Fig. 1. Implementation was done in Python using the TensorFlow library (Google, Mountain View, CA). Training and inference were performed on a Titan XP (NVIDIA, Santa Clara, CA).

### III. RESULTS

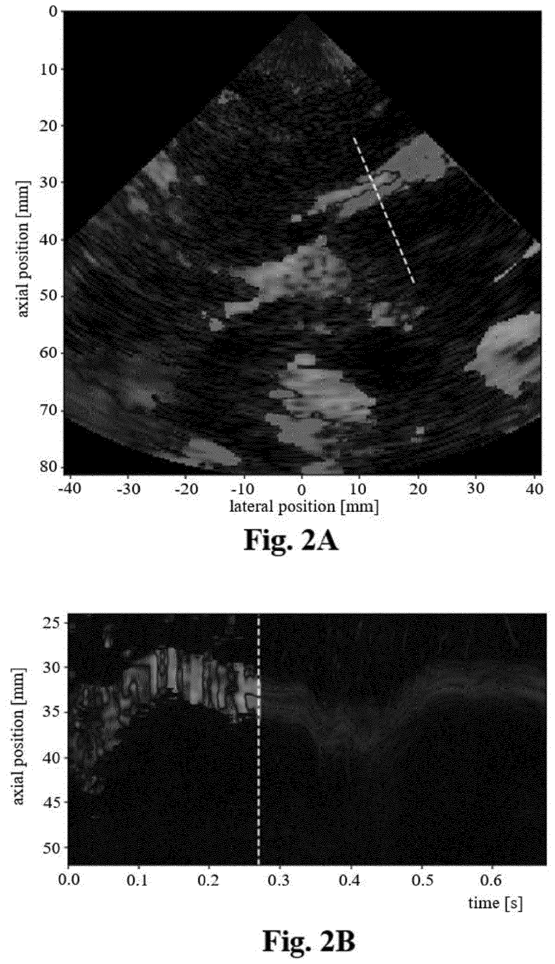

The invention was evaluated on high-frame-rate intra-cardiac ultrasound data obtained with an ICE catheter in combination with a Verasonics Vantage system, described in the data acquisition section. An example of ascertained tissue velocities is given in Figs. 2A and 2B. Fig. 2A shows tissue velocity estimates in the radial direction across space at a specific time instance, whereas Fig. 2B shows tissue velocity estimates in the radial direction along a specific angular line (indicated by dashed line in Fig. 2A) over time, ascertained by using the exemplary neural network described in previous sections.

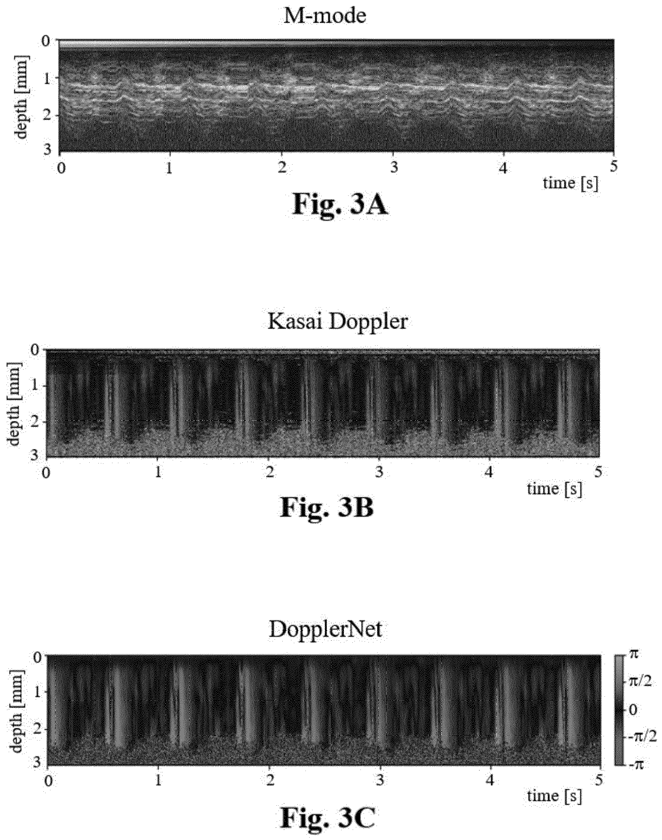

In Figs. 3A to 3C, an example is shown, based on ultrasound data collected by an 8F catheter with four ultrasound transducers integrated into its distal portion. Fig. 3A shows the envelope-detected motion-mode image of left-atrial cardiac tissue, Fig. 3B shows standard Kasai axial velocity estimates within the tissue according to Fig. 3A, and Fig. 3C shows ascertained velocities for various depths of the tissue by using neural network, corresponding to Fig. 3A. The result in Fig. 3C, herein also called as Doppler-Net result, qualitatively displays good agreement with Kasai's method, with significant improvement with respect to lower noise levels and reduced artifacts. Inference is fast, recovering about 20000 Doppler lines per second with GPU acceleration.
It is to be noted that in Fig. 3C there are reduced artifacts with respect to Fig. 3B, particularly the reduction of ring-down of the transducer signal at the top of the image, which enables easier tissue front-wall detection, and also the clearer demarcation of the back-wall of the tissue at the lower part of the image, between the depth of 2 to 3 mm. Furthermore, there is significant reduction of noise levels in the velocities ascertained throughout the thickness of the tissue, which is beneficial for detection of progression of change of velocities in the depth of the tissue upon ablation treatment.

It has been demonstrated that deep neural networks can be used to perform Doppler imaging from compressed acquisitions, by efficiently encoding relevant information into a latent feature space and decoding this as Doppler estimates. By tweaking the convolution strides, down-sampling factors, and the number of (feature) channels in the encoder network, data compression rates between 32:1 - 128:1 can easily be reached; wherein higher rates lead to an increased degree of spatial smoothness and, interestingly, significant noise suppression. The latter is a direct consequence of the inability of expressing signals with such high entropy in the compact basis of the encoder. The achievable compression rates for 2D and 3D ultrasound data is even higher, as there is more spatiotemporal redundancy to exploit.

The ability to perform Doppler using a neural network opens up new possibilities for learning dedicated data compression schemes to reduce the bandwidth for probe-scanner communication.

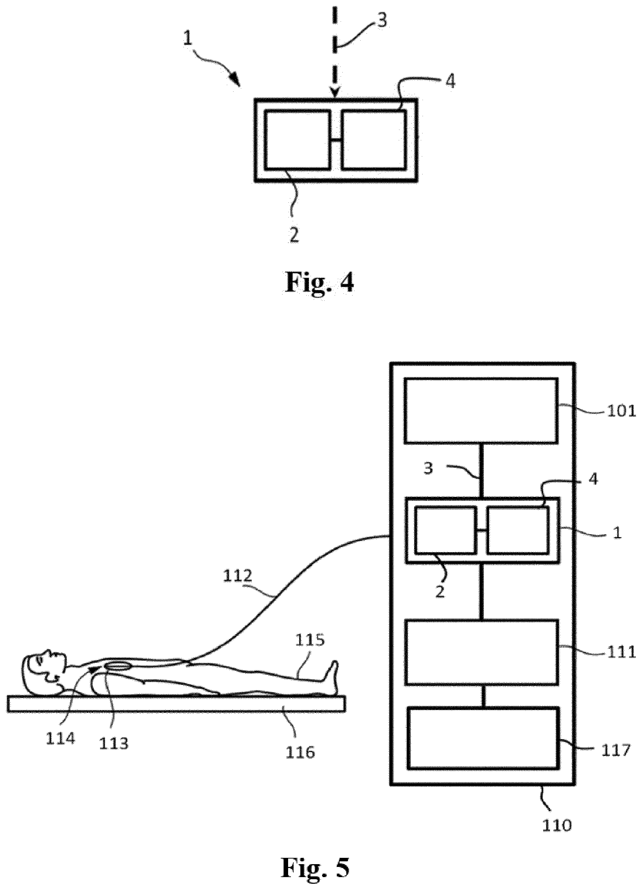

With edge-computing and small neuro ICs making its way into consumer electronics, similar strategies in the design of ultrasound ASICs can be employed. The encoder may be located at or near a distal transducer of the ultrasound device, and the decoder may be located on a remote processor. The remote processor may be integrated into a patient interface module, which transmits the decoded information to a console or directly to a display after further processing. The patient interface module may be located at and integrated into the proximal portion of an intravascular or interventional sensing device (e.g. the energy application device 112 in Fig. 5) and the decoded and processed signals can be sent to a console (e.g. apparatus 1 in Fig. 5) through wired or wireless connection. Some or all of the parameters in said encoder can be quantized, in particular quantized to 1 (binary), 2, 3 or 4 bits. This is a relevant to reduce the memory footprint and therewith power consumption at the distal transducer.
In a preferred embodiment the encoder is designed such that it compresses input data, which in terms of architecture means that the number of input nodes/neurons is larger than the number of output nodes/neurons.
Digitization in the tip of catheter-based transducers can however pose challenges due to generation of heat, which can be alleviated by cooling the distal tip of the catheter, e.g. irrigated catheters with saline, due to which the temperature of the distal catheter tip can be maintained at the desired temperature.

Another application of learning Doppler functionality with neural networks is its use for optimal Doppler-related feature extraction for the purpose of tissue classification. In this context, the preconditioning parts of large deep neural networks aims at performing this characterization task to fulfil physiologically meaningful functionality, such as tissue Doppler and associated strain rate assessment. With this physiology-driven initialization, the network can then further refine its features to achieve optimal classification. The disclosed invention is applicable in the system and methods disclosed in WO 2016/078992 A1, which is hereby incorporated by reference in its entirety for all it teaches and for all purposes.

Fig. 4 shows schematically and exemplarily an embodiment of an apparatus 1 comprising a signal processor 2 for processing ultrasound measurement signals 3 from a tissue according to the data acquisition disclosed above, and a rendering device 4 coupled to the processor 2 for rendering the ultrasound representation, and rendering the representation of the ascertained velocities at various depths of the tissue.

In yet another embodiment schematically shown in Fig. 5, the ultrasound measurement arrangement 101 can be integrated into a system 110 for delivering energy to the tissue during ultrasound measurement. The system comprises an energy source 111 connected to an energy application device 112 for applying energy to the heart 114 tissue of a patient 115 supported by an operation table 116, the ultrasound measurement arrangement 101, a measurement unit 117 for measuring the electrical activity of the heart and the apparatus 1 for visualization of a property of the tissue. The main benefit of such a system is that the apparatus 1 can visualize not only the property of the tissue but also the property change of the tissue upon energy application to the tissue by an energy application device. The ultrasound transducers of the ultrasound measurement arrangement can be integrated into the distal tip 113 of the energy application device 112 (e.g. catheter as described in the data acquisition section), enabling ultrasound measurement localized at the site of the energy application to the tissue. By integrating the ultrasound transducers into the energy application device, the alignment difficulties of an ultrasound probe with respect to the energy application device are eliminated, and shadowing or ringing artifacts are avoided in the ultrasound measurement. The ultrasound transducer integrated into the distal tip 113 of the energy application device 112 may be a single-piston piezoelectical transducer, a phased array piezoelectical transducer or a capacitive micro-machined ultrasound transducer (CMUT). Multiple ultrasound transducers may be integrated into the distal tip of the energy application device for providing ultrasound measurements of the heart tissue in multiple directions simultaneously or sequentially.

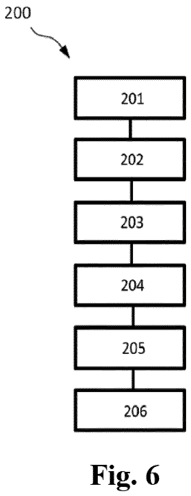

Fig. 6 shows schematically and exemplarily a diagram 200 of the functional use of the system 110. Ultrasound measurement starts in step 201, which can serve initially for positioning the distal tip 113 of the energy application device 112 with respect to the heart 114 tissue and then for ultrasound measurement on the tissue. The system may start measuring and recording the electrical activity of the heart with the measurement unit 117 connected to sensor electrodes integrated into the distal tip of the energy application device and through the electrodes positioned and fixed on the surface of the body of the living being.

In step 202 the signal processor 2 processes ultrasound measurement signals transferred via wired or wireless connection from the ultrasound measurement arrangement 101.

In step 203 a representation is rendered by the rendering device 4 coupled to the processor, representing values of tissue velocity and/or the strain rate. Alternatively, when magnification of the time scale for the representation is selected, than the representation of the electrical activity of the heart is rendered together with the representation of the mechanical interaction of the energy application device with the heart tissue, forming a combined image.

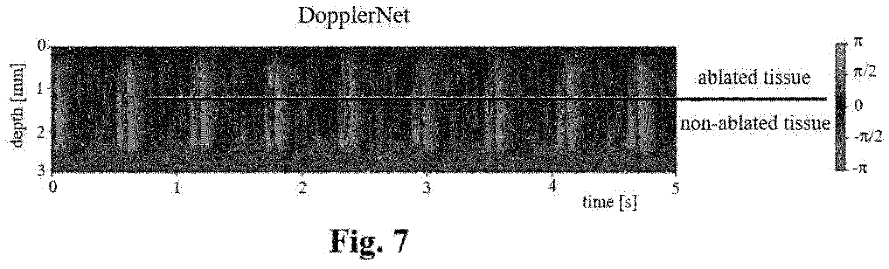

In step 204 the energy application to the tissue starts. The energy is delivered to the tissue through a distal tip 113 of the energy application device 112 connected to the energy source 111 of the system 110. During application of energy to the tissue, the property of the tissue in the representation changes progressively, e.g. the progression of change of velocities in the depth of the tissue in Fig. 7.

In step 205 the processor detects that the progression of change of velocities in the depth of the tissue reached the back-wall of the tissue. When that occurs, the change of the tissue property upon energy application is completed throughout the entire spatial dimension defining the thickness of the tissue, and the energy application can be terminated.

Alternatively, a depth threshold can be regarded as endpoint for the termination of energy application to the tissue, wherein the depth threshold can be defined through a user interface with a computer program controlling the processor.

When the conditions in step 205 are fulfilled, the processor sends a signal to the energy source 111, and in step 206 the energy source discontinues transmission of energy to the energy application device upon receiving the signal.

A computer program element can be provided for controlling the processing apparatus 1, which when being executed by a processor 2 or a processing unit, is adapted to perform the steps according to the method 200. A computer readable medium having stored the program element thereon can also be provided. A computer program may be stored/distributed on a suitable medium, such as an optical storage medium or a solid-state medium, supplied together with or as part of other hardware, but may also be distributed in other forms, such as via the internet or other wired or wireless telecommunication systems.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A system for ascertaining a property of a tissue, comprising a processor (2) configured to:
receive a plurality of radiofrequency or in-phase and quadrature (IQ) demodulated signals (3), wherein said plurality of signals are indicative of reflected echoes from various depths of the tissue over time;
ascertain velocities or strain rate values for the various depths of the tissue from said plurality of signals by using a neural network.

2. The system according to claim 1, wherein said neural network comprises:
an encoder, which compresses said plurality of signals into encoded data;
a decoder, which decodes the encoded data into velocities or strain rate values.

3. The system according to claim 2, further comprising an ultrasound device and wherein said encoder is located at or near a distal transducer of the ultrasound device, and said decoder is located on a remote processor.

4. The system according to claim 3, wherein the remote processor is integrated in a patient interface module.

5. The system according to any of the claims 2 to 4, wherein some or all of the parameters in said encoder are quantized, in particular quantized to 1, 2, 3 or 4 bits.

6. The system according to any of the preceding claims, wherein said neural network comprises:
a plurality of multiplication operations with weights, on said plurality of signals;
nonlinear functions following each said multiplication operation.

7. The system according to any of the claims 1 to 5, wherein said neural network comprises:
a plurality of convolution operations with convolution kernels, on said plurality of signals;
nonlinear functions following each said convolution operation.

8. The system according to any of the preceding claims, wherein the processor is further configured to ascertain strain rate values from said velocities.

9. The system according to any of the preceding claims, wherein the output of said encoder has fewer nodes than the input of said encoder.

10. The system of any preceding claims, comprising an energy source (111) connected to an energy application device (112) for applying energy to a tissue so as to change the property of the tissue, an ultrasound measurement arrangement (101), wherein the system (110) is arranged to discontinue transmission of energy from the energy source (111) to the energy application device (112) in response to a detected progression of change of velocities and/or strain rate values in the depth of the tissue.

11. A method of ascertaining a property of a tissue, the method comprising:
receiving a plurality of radiofrequency or in-phase and quadrature (IQ) demodulated signals, wherein said plurality of signals are indicative of reflected echoes from various depths of the tissue over time;
ascertaining velocities or strain rate values at various depths of the tissue from said signals by using a neural network.

12. The method according to claim 11, wherein said neural network comprises:
an encoder, compressing said plurality of signals into encoded data;
a decoder, decoding the encoded data into velocities or strain rate values.

13. The method according to claim 12, further comprising:
locating said encoder at or near a distal transducer of an ultrasound device, and
locating said decoder on a remote processor.

14. The method according to any of the claims 11 to 13, further comprising ascertaining strain rate values from said velocities.

15. The method according to any of the claims 11 to 14, further comprising:
applying energy to a tissue so as to change the property of the tissue;
discontinuing transmission of energy to the tissue in response to a detected progression of change of velocities and/or strain rate values in the depth of the tissue.
